(19) Europäisches Patentamt
European Patent Office
Office européen des brevets

(11) Veröffentlichungsnummer : **0 040 745**
**B1**

(12) EUROPÄISCHE PATENTSCHRIFT

(45) Veröffentlichungstag der Patentschrift : **26.09.84**

(51) Int. Cl.³ : **C 07 D249/06**

(21) Anmeldenummer : **81103588.0**

(22) Anmeldetag : **11.05.81**

# (54) Verfahren zur Herstellung von v-Triazolverbindungen.

(30) Priorität : **22.05.80 DE 3019521**

(43) Veröffentlichungstag der Anmeldung : **02.12.81 Patentblatt 81/48**

(45) Bekanntmachung des Hinweises auf die Patenterteilung : **26.09.84 Patentblatt 84/39**

(84) Benannte Vertragsstaaten : **CH DE FR GB IT LI**

(56) Entgegenhaltungen :
**DE-A- 1 670 914**
**DE-A- 2 210 261**
**DE-A- 2 242 784**
**DE-A- 2 254 300**
**DE-A- 2 423 091**

(73) Patentinhaber : **BAYER AG**
**Konzernverwaltung RP Patentabteilung**
**D-5090 Leverkusen 1 Bayerwerk (DE)**

(72) Erfinder : **Dorlars, Alfons, Dr.**
**Mozartstrasse 32**
**D-5090 Leverkusen (DE)**

**EP 0 040 745 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung ist ein verbessertes Verfahren zur Herstellung von wasserlöslichen 2-Aryl-v-triazolverbindungen durch Cyclodehydratisierung entsprechender $\alpha$-Oximinoarylhydrazone.

Die Herstellung der 2-Aryl-v-triazole, welche unter anderem technisch wertvolle UV-Absorber und optische Aufheller sind, erfolgt bislang ausschließlich durch Einwirkung von Acylierungsmitteln, wie Säureanhydriden, Säurehalogeniden, Harnstoff, Isocyanaten, auf geeignete Oximinohydrazone (vgl. DE-A-2 338 881, DE-B-2 746 000, DE-A-1 670 914, US-A-3 666 758, US-A-3 947 412, US-A-3 965 094 und GB-A-1 405 218), bzw. durch bloßes Erwärmen der vorher acylierten Oximinohydrazone (vgl. DE-A-2 423 091).

Diese an sich vielfach bewährten Verfahren weisen indessen den Nachteil auf, daß die im übrigen relativ teuren Cyclisierungsmittel das Abwasser stark belasten. Es wurde nun überraschenderweise gefunden, daß man wasserlösliche 2-Aryl-v-triazole der Formel I

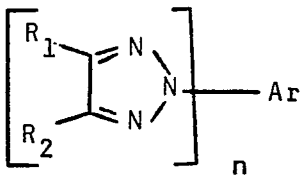

(I)

worin

$R_1$ und $R_2$ unabhängig voneinander für gegebenenfalls substituiertes Alkyl oder für einen gegebenenfalls substituierten Rest Ar und

n für die Zahlen 1 oder 2 stehen, wobei

Ar einen mindestens eine Sulfonsäure-, Phosphonsäure- oder Benzoldisulfimidgruppe enthaltenden Arylrest aus der Benzol-, Naphthalin-, Diphenylmethan-, Diphenylethan-, Stilben- oder Tolanreihe bedeutet,

auf einfache Weise und in überraschend guten Ausbeuten durch Cyclodehydratisierung entsprechender $\alpha$-Oximinoarylhydrazone erhält, wenn man den Ringschluß bei 120-200 °C in Wasser oder vorwiegend wäßrigen Medien in Abwesenheit der üblichen Cyclisierungsmittel — gegebenenfalls jedoch in Gegenwart von Cu-I-salzen — herbeiführt.

Geeignete Alkylreste in der Formel I sind insbesondere solche mit 1-4 C-Atomen.

Geeignete Arylreste $R_1$, $R_2$ und Ar sind insbesondere Phenylreste, die durch übliche, nichtchromophore Substituenten, wie Halogen, $C_1$-$C_4$-Alkyl, $C_1$-$C_4$-Alkoxy oder Phenyl, substituiert sein können.

Unter « Halogen » wird F, Br und besonders Cl verstanden.

Geeignete Oximinohydrazone sind solche der Formel II

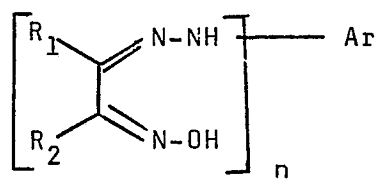

(II)

Die Cyclodehydratisierung dieser Verbindungen erfolgt zweckmäßigerweise unter Rühren in geeigneten Gefäßen (z. B. Niederdruckkesseln) bei pH-Werten von 4-8.

Zur Einstellung dieses pH-Bereichs kann die Anwesenheit von Puffersubstanzen vorteilhaft sein.

Neben Cu-I-salzen zeigen auch (metallisches) Kupfer und Cu-II-salze sowie andere Schwermetalle bzw. deren Salze eine beschleunigte bzw. ausbeutesteigernde Wirkung.

Das Ende der Reaktion läßt sich leicht analytisch bestimmen, z. B. mittels Dünnschichtchromatographie einer entnommenen Probe. Die entstandenen 4,5-disubstituierten v-Triazolylsäuren bzw. deren Salze lassen sich nach Abkühlen der Reaktionsmischung in einfacher Weise isolieren, z. B. durch Aussalzen.

Die als Ausgangsmaterialien verwendeten Oximinohydrazone der Formel II brauchen zur Durchführung des erfindungsgemäßen Verfahrens nicht isoliert werden. In einer besonders vorteilhaften Ausführungsform dieses Verfahrens wird vielmehr das Reaktionsgemisch der Kondensation der Oximinoketone der Formel III

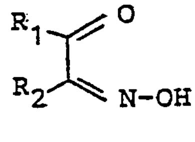

(III)

und der Arylhydrazine IV

$$Ar{-}NH{-}NH_2 \qquad (IV)$$

ohne Zwischenisolierung — gegebenenfalls nach Abdestillieren eventuell vorhandener leicht flüchtiger Lösungsmittelanteile und nach Zusatz von Puffersalzen und Kupferkatalysatoren — auf die erforderliche Reaktionstemperatur erhitzt.

Der bevorzugte Temperaturbereich liegt dabei bei 140-165 °C.

In diesem Intervall ist die Umsetzung zum Triazol in den meisten Fällen nach 5-6 Stunden beendet.

Beispiel 1

3-[4-Ethyl-5-methyl-v-triazolyl-(2)]-benzolsulfonsäure (Na-Salz).

In einen 3 1-Niederdruck-Kessel werden in vorgelegte 1,5 l Wasser bei Raumtemperatur unter Rühren 570 g (3 Mol) Phenylhydrazin-3-sulfonsäure und 360 g (3,1 Mol) Oximinodiethylketon eingetragen. Durch Zutropfen von 40 %iger Natronlauge stellt man auf einen pH-Wert von 5,0-5,5 ein, wobei man gleichzeitig allmählich auf 55° heizt. Dabei verschwindet die Phenylhydrazinsulfonsäure und das entstandene Oximinohydrazon kristallisiert aus. Unter diesen Bedingungen ist die Kondensation nach etwa 1-2 Stunden beendet ; man stellt dann mit Natronlauge auf pH 7 ein (Gesamtverbrauch ca. 200 ml 40 %ige Natronlauge ; Volumen ca. 2 l). Darauf gibt man 5 g Kupfer-I-chlorid hinzu, schließt den Kessel, heizt innerhalb von einer Stunde auf 150° und rührt weitere 5-6 Stunden bei dieser Temperatur. Schließlich läßt man auf 95-98° abkühlen und klärt nach Einrühren von 15 g Adsorptionskohle durch ein Saugfilter ; der pH-Wert des Filtrates ist nahezu unverändert geblieben (pH 7). Man engt die Lösung auf ca. 1,5 l ein, rührt 80 g Kochsalz ein und kühlt allmählich auf 0-5°. Das als dicker Kristallbrei ausgeschiedene Natriumsalz der 3-[4-Ethyl-5-methyl-v-triazolyl-(2)]-benzolsulfonsäure wird abgesaugt, gepreßt und bei 110° getrocknet. Ausbeute : 782 g 95 %iges Na-Salz (86 % der Theorie) als farblose Kristalle.

Mit ähnlichen Ausbeuten und in analoger Weise lassen sich die in der folgenden Tabelle aufgeführten Triazole herstellen.

Tabelle

Triazole der Formel

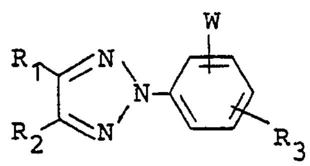

| Beispiel | $R_1$ | $R_2$ | $R_3$ | W |
|---|---|---|---|---|
| 2 | $C_2H_5$ | $CH_3$ | H | 3-$SO_3Na$ |
| 3 | $C_2H_5$ | $CH_3$ | H | 4-$SO_3Na$ |
| 4 | 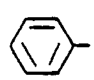 | $CH_3$ | H | 3-$SO_3Na$ |
| 5 | 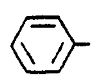 | $CH_3$ | H | 2-$SO_3Na$ |
| 6 | 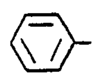 | $C_2H_5$ | H | 3-$SO_3Na$ |
| 7 | 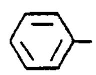 | $CH_3$ | 4-$CH_3$ | 3-$SO_3Na$ |
| 8 | 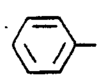 | 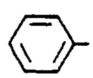 | H | 3-$SO_3Na$ |
| 9 | 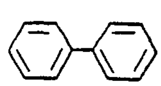 | $CH_3$ | H | 3-$SO_3Na$ |

Tabelle (Fortsetzung)

| Beispiel | $R_1$ | $R_2$ | $R_3$ | W |
|---|---|---|---|---|
| 10 | 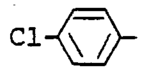 | $CH_3$ | H | $3-SO_3Na$ |
| 11 | 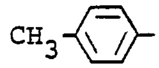 | $CH_3$ | H | $3-SO_3Na$ |
| 12 | 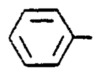 | $CH_3$ | H | $4-PO_3Na_2$ |
| 13 | $C_2H_5$ | $CH_3$ | H | $3-SO_3Na-SO_2-$ 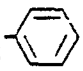 |
| 14 | 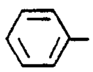 | $CH_3$ | 4-Cl | $3-SO_3Na$ |
| 15 | $C_3H_7$ | $C_2H_5$ | H | $3-SO_3Na$ |
| 16 | $CH_3$ | $CH_3$ | H | $3-SO_3Na$ |

Beispiel 17

4,4'-Bis-[4-phenyl-5-methyl-v-triazolyl-(2)]-stilben-2,2-disulfonsäure (Na-Salz).

80 g 4,4'-Dihydrazinostilben-2,2'-disulfonsäure, suspendiert in 300 ml Wasser, werden mit einer Lösung von 70 g Oximinopropiophenon in 300 ml Methanol versetzt. Man erwärmt die Mischung allmählich auf 65° und hält den pH-Wert durch Einrühren von Natriumacetat auf 4,8-5,0 (ca. 50 g). Nach etwa 3 1/2 Stunden ist die Kondensation beendet ; man destilliert dann den größten Teil des Methanols ab und gibt 500 ml kaltes Wasser hinzu, sowie 100 ml konzentrierte Kochsalzlösung. Das ausgeschiedene Kondensationsprodukt wird abfiltriert, in 1,3 l Wasser suspendiert, die Suspension mit Natriumacetat auf pH 7,5 eingestellt und mit 2 g Kupfer-I-chlorid versetzt. Man heizt darauf unter Rühren auf 155° und hält diese Temperatur weitere 5 Stunden. Darauf läßt man auf 80° abkühlen, versetzt mit 60 ml konzentrierter Kochsalzlösung und saugt das entstandene bis-triazolylstilben-disulfonsaure Salz ab, das man durch Umlösen aus wäßrigem Methylglykol reinigt und trocknet. Man erhält 99 g (71 % der Theorie) in Form gelber Kristalle.

## Anspruch

Verfahren zur Herstellung von wasserlöslichen Aryl-v-triazolen der Formel I

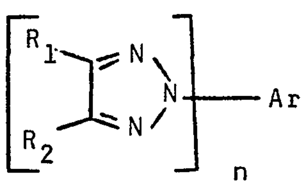

(I)

worin

$R_1$ und $R_2$ unabhängig voneinander für gegebenenfalls substituiertes Alkyl oder für einen gegebenenfalls substituierten Rest Ar und

n für die Zahlen 1 oder 2 stehen, wobei

Ar einen mindestens eine Sulfonsäure-, Phosphonsäure- oder Benzoldisulfimidgruppe enthaltenden Arylrest aus der Benzol-, Naphthalin-, Diphenylmethan-, Diphenylethan-, Stilben-oder Tolanreihe bedeutet,

durch Cyclodehydratisierung entsprechender α-Oximinihydrazone, dadurch gekennzeichnet, daß man den Ringschluß bei 120-200 °C in Wasser oder vorwiegend wäßrigen Medien in Abwesenheit der üblichen Cyclisierungsmittel — gegebenenfalls jedoch in Gegenwart von Cu-I-salzen — herbeiführt.

**Claim**

Process for the preparation of water-soluble aryl-v-triazoles of the formula I

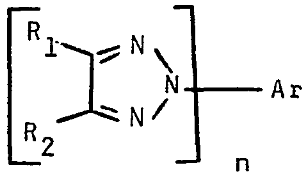

(I)

wherein

$R_1$ and $R_2$ independently of one another represent optionally substituted alkyl or an optionally substituted radical Ar and

n represents the number 1 or 2,

Ar denoting an aryl radical which is from the benzene, naphthalene, diphenylmethane, diphenylethane, stilbene or tolane series and contains at least one sulphonic acid group, phosphonic acid group or benzene disulphimide group,

by cyclodehydration of corresponding α-oximinohydrazones, characterised in that cyclisation is brought about at 120-200 °C in water or predominantly aqueous media in the absence of the customary cyclising agents — but, if appropriate, in the presence of Cu-I salts.

**Revendication**

Procédé pour la fabrication d'aryl-v-triazoles solubles dans l'eau de formule I

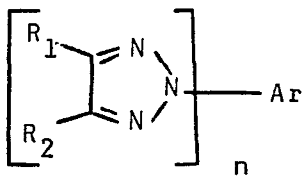

(I)

dans laquelle

$R_1$ et $R_2$ représentent indépendamment l'un de l'autre un reste alkyle éventuellement substitué ou un reste Ar éventuellement substitué, et

n représente le nombre 1 ou 2,

le reste Ar étant un reste aryle de la série du benzène, du naphtalène, du diphénylméthane, du diphényléthane, du stilbène ou du tolane contenant au moins un groupe acide sulfonique, acide phosphonique ou benzène disulfimide,

par cyclodéshydratation d'α-oximino-arylhydrazones, caractérisé en ce qu'on provoque la cyclisation à 120-200 °C dans l'eau ou des milieux essentiellement aqueux en l'absence des agents cyclisants habituels, mais éventuellement en présence de sels de Cu (I).